# EUROPÄISCHE PATENTSCHRIFT

(11) **EP 2 011 475 B1**
(45) Veröffentlichungstag und Bekanntmachung des Hinweises auf die Patenterteilung: **02.03.2011**
(21) Anmeldenummer: 08011336.8
(22) Anmeldetag: 23.06.2008
(51) Int. Cl.: A61K 8/34, A61K 8/365, A61K 8/46, A61K 8/49, A61K 8/81, A61Q 17/00, A01N 31/02

(54) **Antiseptisches Hautfärbemittel**
Antiseptic skin dye
Produit de coloration de la peau antiseptique

(30) Priorität: 29.06.2007 DE 102007030416
(43) Veröffentlichungstag der Anmeldung: 07.01.2009
(73) Patentinhaber: Bode Chemie GmbH, 22525 Hamburg (DE)
(72) Erfinder: Stengele, Michael, Dr., 20257 Hamburg (DE); Rudolf, Marco, 25469 Halstenbek (DE); Schulte, Elke, 22047 Hamburg (DE); Ostermeyer, Christiane, 22559 Hamburg (DE); Kampf, Günter, Dr., 20257 Hamburg (DE); Feil, Yvonne, 25436 Tornesch (DE); Mueller, Kai-Martin, 22527 Hamburg (DE); Dabek, Sven, Dr., 22523 Hamburg (DE)
(74) Vertreter: Kossak, Sabine

(56) Entgegenhaltungen:
- EP-A- 1 044 686
- WO-A-02/091832
- DE-A1- 4 137 548
- US-A- 5 244 666
- US-B1- 6 333 039

## Beschreibung

Gegenstand der Erfindung ist ein antiseptisches Hautfärbemittel, das einen oder mehrere alkoholische Wirkstoffe, ggf. eine oder mehrere hautverträgliche, organische Säuren, einen oder mehrere Farbstoffe, ggf. weitere Hilfsstoffe, ggf. weitere Wirkstoffe und Wasser enthält, wobei sich die Komponenten zu 100 Gew.-% ergänzen.

In verschiedenen Bereichen der Medizin ist es notwendig, kleinere oder größere Hautareale anzufärben, beispielsweise vor einer Operation, Injektion oder Punktion. Die dabei verwendeten Färbelösungen können zusätzlich eine desinfizierende Wirkung haben und durch die Anfärbung der Haut ist es dem medizinischen Personal möglich, sicherzustellen, dass der gesamte Bereich desinfiziert wurde. Auf dem Markt sind verschiedene Produkte mit Jod oder Jodderivaten auf nicht alkoholischer Basis erhältlich. Es hat sich jedoch gezeigt, dass durch das enthaltene Jod diese Produkte häufig schlechter verträglich sind als vergleichbare ungefärbte Produkte.

Alkoholische Hautfärbemittel, mit denen das behandelte Hautareal farblich markiert werden soll, enthalten in der Regel einen oder mehrere Azo-Farbstoffe. Diese Gruppe der Farbstoffe wird allerdings aus toxikologischen Gründen zunehmend kritisch bewertet. Schon 2001 wurde in den technischen Regel für Gefahrstoffe 614 ("Verwendungsbeschränkung für Azo-Farbstoffe, die in krebserzeugende aromatische Amine gespalten werden können") beschrieben, dass manche dieser Azo-Farbstoffe nur beschränkt eingesetzt werden sollten, bzw. nach Möglichkeit auf ihre Nutzung vollständig verzichtet werden sollte. Auch bei Azo-Farbstoffen hat sich gezeigt, dass die Verträglichkeit auf der Haut für die vergleichbaren ungefärbten Produkte deutlich besser ist.

Aus der EP 1 044 686 A ist ein antiseptisches Mittel, welches Alkohol, Wasser und Chlorhexidin enthält, bekannt, welches sich insbesondere zur chirurgischen Schnelldesinfektion eignen soll. Die farbige alkoholische Lösung gemäß der EP 1 044 686 A ist nicht zur Anfärbung des Operationsfeldes geeignet. Die Färbeleistung diverser Farbstoffe ist beschrieben, wobei auf die Stabilitätsprobleme colorierter Lösungen hingewiesen und hervorgehoben ist, dass trotz des Versuches, stabile Lösungen zu erhalten, eine gewisse Kristallisation der Farbstoffe nicht verhindert werden kann.

Die US 6,333,039 B1 beschreibt eine opake Zusammensetzung, die hautdesinfizierend wirkt. Bestandteile dieser Zusammensetzung sind mindestens 50 Gew.-% reiner Alkohol mit 1 bis 4 Kohlenstoffatomen, 0,1 bis 5 Gew.-% mindestens eines Agenzes, welches den Feuchtigkeitsgehalt der Haut reguliert, ein Verdickungsmittel sowie Wasser. Eine gewisse optische Änderung der mit der Lösung behandelten Haut wird dadurch erreicht, dass das den Feuchtigkeitshaushalt der Haut regulierende Agenz gleichzeitig eine Opazität der Zusammensetzung bewirkt. Der Einsatz geeigneter Farbstoffe, um ein deutliches Anfärben der Haut zu bewirken, ist jedoch nicht beschrieben.

Die DE 41 37 548 A1 offenbart eine antimikrobielle Wirkstoffkombination auf der Basis von Acridinfarbstoffen, welche optional diverse andere Bestandteile, z.B. Milchsäure oder Chlorhexidin, enthält.

Die US 5,244,666 beschreibt ein chirurgisches Desinfektionsmittel, welches eine quarternäre Ammoniumverbindung, eine phenolische Verbindung, einen Weichmacher, ein Detergenz, ein Wundheilmittel und ein viskositätssteigerndes und Emulgator-Agenz enthält. Dieses umfasst ein synthetisches Phospholipid. Als Farbstoff wird Chinolingelb eingesetzt.

In der WO 02/091832 A1 ist ein System aus zwei Zusammensetzungen beschrieben, welche unter Vermischung ein wässriges Desinfektionsmittel ergeben. Die erste Zusammensetzung enthält ein Chlorid. In der zweiten Zusammensetzung sind eine Säure und optional ein oxidierbarer Farbstoff enthalten. Diverse handelsübliche Farbstoffe sind als geeignete Farbstoffe genannt. Daneben enthält mindestens eine der Zusammensetzungen ein alpha-Olefin-Sulfonat.

In keiner der Schriften ist ein physikalisch und chemisch stabiles antiseptisches Hautfärbemittel zur Einfärbung von Hautarealen beim Menschen beschrieben, das die Anforderungen eines guten Farbverhaltens bei einer guten Verträglichkeit und Entfernbarkeit zeigt und seine Wirksamkeit bereits bei sehr kurzer Einwirkzeit entfaltet.

Aufgabe der vorliegenden Erfindung ist es daher, ein antiseptisches Hautfärbemittel bereitzustellen, das ein gutes Färbeverhalten auf der Haut zeigt, einen oder mehrere in alkoholischer Lösung stabile Farbstoffe enthält und die gleiche Verträglichkeit, wie ein vergleichbares ungefärbtes Produkt aufweist. Der im Hautfärbemittel enthaltene Farbstoff muss eine gute chemische und physikalische Stabilität aufweisen und bei verschiedenen pH-Werten eine stabile Lösung bilden. Zudem muss sich das Hautfärbemittel gut von der Haut und verschiedenen Materialien, z.B. Wand oder Boden, entfernen lassen.

Die Aufgabe wird erfindungsgemäß gelöst durch ein antiseptisches Hautfärbemittel gemäß Anspruch 1.

Weitere Ausführungsformen sind Gegenstand der Unteransprüche oder nachfolgend beschrieben.

In dem erfindungsgemäßen Hautfärbemittel werden die in der Arzneimittelverordnung genannten synthetischen organischen Farbstoffe Chinolingelb (E 104), 2-(1,3-Dioxo-2-indanyl)chinolindisulfonsäure und deren Dinatriumsalz), Patentblau V (E 131, α-(4-Diethylaminophenyl)-α-(4-Diethyliminio-2,5-cyclo-hexadien-1-yliden)-5-hydroxy-4-sulfo-o-toluolsulfonat, Calciumsalz), Indigotin (E 132) oder Brilliantgrün BS (E 142, 1-(α-(4-Dimethyliminio-2,5-cyclohexadien-1-yliden)-4-dimethylamniobenzyl)-2-hydroxy-6-sulfo-3-naphthalinsulfonat, Natriumsalz) eingesetzt. Überraschenderweise hat sich gezeigt, dass die erfindungsgemäß eingesetzten Farbstoffe auch in hochalkoholischen Produkten stabil sind. Die Farbstoffe zeigen eine gute chemische und physikalische Stabilität. Auch bei unterschiedlichen pH-Werten konnte eine stabile Lösung erhalten werden. Dieses zeichnet die erfindungsgemäß eingesetzten synthetischen organischen Farbstoffe gegenüber entsprechend natürlichen oder naturidentisch synthetischen Farbstoffen aus. Bei Experimenten mit natürlichen und naturidentisch synthetischen Farbstoffen in alkoholischen Lösungen zeigten sich diese vielfach als nicht stabil und es kam zu Ausflockungen bzw. einer unerwünschten Farbveränderung während der Lagerung. Damit ein Einsatz als Hautfärbemittel möglich ist, müssen die Farbstoffe eine stabile Lösung bilden und gut in Lösung zu bringen sein. Bei verschiedenen natürlichen und naturidentisch synthetischen Farbstoffen wurde beobachtet, dass diese ausfallen oder nicht restfrei lösbar sind. Zudem schwankt die Löslichkeit und die Stabilität der Lösung mit dem pH-Wert. Im Gegensatz dazu sind die erfindungsgemäß eingesetzten synthetischen organischen Farbstoffe klar löslich und bieten eine Lagerstabilität von Monaten, ohne dass der Farbstoff ausfällt.

Die erfindungsgemäß eingesetzten Farbstoffe bieten zudem ein gutes Färbeverhalten auf der Haut, was sie ebenfalls gegenüber natürlichen und naturidentisch synthetischen Farbstoffen auszeichnet, die in alkoholischen Produkten meist ein ungenügendes Färbeverhalten auf der Haut zeigen. Außerdem ist bei dem erfindungsgemä-ßen Hautfärbemittel eine gute Entfernung der Farbe von der Hautpartie oder verschiedenen Materialien, möglich. Das erfindungsgemäße Hautfärbemittel wird deshalb als gut materialverträglich bezeichnet, da es sich leicht von verschiedenen Materialien, d.h. beispielsweise Böden, Wänden, Instrumenten und Krankhauseinrichtungen entfernen lässt. Die Entfernung des erfindungsgemäßen Hautfärbemittels ist dabei teilweise bereits durch Abwaschen mit Wasser und Seifen möglich und es konnte eine deutlich leichtere Entfernung der Farbstoffe als bei vergleichbaren Produkten mit Azo-Farbstoffen beobachtet werden. Dies gilt sowohl für die Entfernung des Hautfärbemittels von der Haut als auch von verschiedenen Materialien, wie Böden oder Keramik.

Gegenüber den Azo-Farbstoffen bieten die erfindungsgemäß eingesetzten Farbstoffe den Vorteil, dass sie toxikologisch unbedenklich sind. Überraschenderweise konnte zudem keine Verschlechterung der Hautverträglichkeit beim Vergleich des erfindungsgemäßen Hautfärbemittels mit dem vergleichbaren ungefärbten Produkt beobachtet werden. Da dieses bei den bisher bekannten Azo-Farbstoffen der Fall war, war dieses Verhalten völlig unerwartet.

Das erfindungsgemäße Hautfärbemittel enthält als alkoholischen Wirkstoff einen Alkohol mit 1 bis 3 Kohlenstoffatomen oder eine Mischung der entsprechenden Alkohole, bevorzugt Monoalkohole. Bevorzugt wird als alkoholischer Wirkstoff Ethanol, 1-Propanol, 2-Propanol oder eine Mischung dieser Alkohole eingesetzt. Der Gesamtalkoholgehalt des alkoholischen Wirkstoffs beträgt in dem erfindungsgemäßen Hautfärbemittel 50 bis 99 Gew.%, bevorzugt 50 bis 95 Gew.-%, weiterhin bevorzugte Mengen sind 50 bis 70 Gew.-%, besonders bevorzugt 50 bis 65 Gew.-%, oder bevorzugt 75 bis 87 Gew.-%. Dieses gilt sowohl wenn Ethanol, 1-Propanol, 2-Propanol als alleiniger alkoholischer Wirkstoff, als auch wenn diese in Mischungen mit den anderen alkoholischen Wirkstoffen eingesetzt werden. Als Ethanol wird dabei Ethanol (99%), der mit 1 % Butanon vergällt ist, eingesetzt.

Wird Ethanol als alleiniger alkoholischer Wirkstoff eingesetzt, so wird Ethanol (99%) bevorzugt in einer Menge von 80 bis 90 Gew.-%, besonders bevorzugt 85,9 Gew.-%, eingesetzt. Für 1-Propanol sind bevorzugte Mengen, die im erfindungsgemäßen Hautfärbemittel eingesetzt werden, 50 bis 60 Gew.-%, besonders bevorzugt 53,5 Gew.-% und für 2-Propanol 60 bis 70 Gew.-%, besonders bevorzugt 63 Gew.-%.

Das erfindungsgemäße Hautfärbemittel enthält ggf. eine oder mehrere hautverträgliche, organische Säuren. Geeignete hautverträgliche Säuren sind beispielsweise Mono-, Di- und Polycarbonsäuren, die ggf. mit einer oder mehreren Hydroxygruppen substituiert sind. Bevorzugt sind die organische(n) Säure(n) Zitronensäure, Ascorbinsäure, Milchsäure oder Pidolsäure (2-Pyrrolidon-5-carbonsäure), besonders bevorzugt Milchsäure. In einer Ausführungsform enthält das erfindungsgemäße Hautfärbemittel als antimikrobielle Wirkstoffe ausschließlich Ethanol, 1-Propanol, 2-Propanol oder Mischungen dieser Stoffe. In einer weiteren Ausführungsform enthält das erfindungsgemäße Hautfärbemittel als Hilfsstoffe weitere mikrobiozid- und/oder viruswirksame Stoffe. Beispielsweise können als weitere antimikrobielle Zusatzstoffe zugesetzt werden:

| **Chemische Klasse** | **Beispiele** |
|---|---|
| Quaternäre Ammoniumverbindungen | Benzalkoniumchlorid, Mecetronium etilsulfat |
| | |
| Guanidine | Polyhexanid, Chlorhexidinsalz |
| | |
| Pyridin-Derivate | Octenidindihydrochlorid, Zink-Pyrithion |
| | |
| Phenol-Derivate | o-Phenylphenol, p-Chlor-m-Kresol, 2-Phenoxyethanol, Phenoxypropanol |

Das erfindungsgemäße Hautfärbemittel enthält ggf. Hilfsstoffe in einer Menge von 0,05 bis 5 Gew.-%. Dabei werden als Hilfsstoffe beispielsweise wasserlösliche und/oder alkohollösliche Polymere zur Verbesserung der Stabilität der Lösung und zur besseren Entfernbarkeit der Farbstoffe von der Haut, Verdickungsmittel, wie Acrylate und deren Derivate, Cellulose und Cellulosederivate oder andere handelsübliche Verdicker, hautverträgliche Tenside oder Emulgatoren zugesetzt. Als Hilfsstoff Polyvinylpyrrolidon (PVP) eingesetzt, das u.a. unter den Handelsnamen Kollidon^{®} 90F erhältlich ist. Dieses wird in einer Menge von 0,05 bis 1 Gew.-% eingesetzt. Der eingesetzte Ethanol kann zudem Vergällungsmittel wie 2-Butanon enthalten.

Das erfindungsgemäße Hautfärbemittel eignet sich zur Einfärbung kleiner und großer Hautareale vor einer chirurgischen oder medizinischen Behandlung, wie beispielsweise vor einer Operation, einer Injektion oder einer Punktion. Die dabei verwendeten Färbelösungen können zusätzliche eine desinfizierende Wirkung haben und durch die Anfärbung der Haut ist es dem medizinischen Personal möglich, sicherzustellen, dass der gesamte Bereich desinfiziert wurde. Die im erfindungsgemäßen Hautfärbemittel enthaltenen Wirkstoffe sorgen zudem dafür, dass eine schnelle und zuverlässige Hautdesinfektion erfolgt, wobei eine Reduzierung der Bakterien, Viren und Pilze erreicht wird. Das erfindungsgemäße Hautfärbemittel kann daher auch als Hautdesinfektionsmittel verwendet werden. Es zeigt eine gute antiseptische Wirkung gegenüber der residenten und transienten Hautflora.

Die Lösungen haben je nach eingesetzten Komponenten einen pH-Wert, der bevorzugt zwischen 2 und 10, besonders bevorzugt zwischen 3 und 9, liegt.

Die Erfindung soll anhand der nachfolgenden Beispiele zusätzlich erläutert werden.

### Beispiele

Es wurden verschiedene erfindungsgemäße Hautfärbemittel hergestellt, die die in der Tabelle 1 angegebene Zusammensetzung in Gewichtsprozent haben. Bei allen Zusammensetzungen wurden stabile Lösungen erhalten und es konnte kein Ausflocken der Farbstoffe beobachtet werden. Die Hautfärbemittel zeigen ein gutes Färbeverhalten auf der Haut. Zudem konnte eine gute Materialverträglichkeit und eine gute Entfernbarkeit des Hautfärbemittels beobachtet werden.

| | A nicht erfindungsgemäß | B | C nicht erfindungsgemäß | D nicht erfindungsgemäß | E |
|---|---|---|---|---|---|
| Ethanol (99%) vergällt mit 1% 2-Butanon | - | 85,9 | - | - | 85,9 |
| 1-Propanol | - | - | - | 8 | - |
| 2-Propanol | 63 | - | 63 | 65 | - |
| E 104 | 0,088 | 0,13 | - | 0,033 | 0,0096 |
| E 131 | - | - | - | - | 0,00124 |
| E 142 | - | - | 0,0044 | - | - |
| PVP | - | 0,5 | - | - | 0,5 |
| Milchsäure (90%) | - | 1 | - | - | 1 |
| Benzalkoniumchlorid | 0,025 | - | 0,025 | 0,025 | - |
| Wasser | ad.100 | ad.100 | ad.100 | ad.100 | ad.100 |

### Prüfung der Verträglichkeit der Hautfärbemittel

### 1. Vergleichsversuch

Es wurde ein Hautfärbemittel mit 85,9 Gew.-% Ethanol (99%) und den Azo-Farbstoffen E 110 und E 151, weiteren Farbstoffen, einer Säure und einem Hilfsstoff hergestellt. Als Vergleichsmischung wurde eine Lösung aus 85,9 Gew.-% Ethanol (99%), einer Säure und Wasser verwendet. Mit diesem Hautfärbemittel wurde die lokale Verträglichkeit mit Hilfe des repetitiven okklusiven Patch-Test gemessen.
- Repetitiver okklusiver Patch-Test (visuelle Bewertung der behandelten Haut)
- 20 Probanden
- 4 x 22 h Behandlung
- Negativkontrolle: 0.9% Kochsalzlösung
- Ergebnis (gefärbte Rezeptur):
   o 13 Probanden ohne Hautreaktion
   o 4 Probanden mit leichter Hautrötung
   o 3 Probanden mit klarem Erythem
   o Summenscore: 21
- Ergebnis (ungefärbte Rezeptur):
   o 14 Probanden ohne Hautreaktion
   o 6 Probanden mit leichter Hautrötung
   o keine Probanden mit klarem Erythem
   o Summenscore: 14

Fazit: Das irritative Potential der farbigen alkoholischen Lösung ist ca. 50% höher als das der ungefärbten Variante, verursacht durch die verwendeten Azofarbstoffe.

### 2. Versuch mit erfindungsgemäßem Hautfärbemittel

Es wurde ein erfindungsgemäßes Hautfärbemittel hergestellt, das 85,9 Gew.-% Ethanol (99%), Milchsäure, Polyvinylpyrrolidon und als Farbstoffe E 104 und E 131 enthielt. Zur Messung der lokalen Verträglichkeit wurde mit 44 Probanden ein repetitiver okklusiver Patch-Test durchgeführt im Vergleich mit der farbstofffreien Rezeptur. Die farbstofffreie Rezeptur enthielt 85,9 Gew.-% Ethanol (99%), eine Säure und Wasser.
o Repetitiver okklusiver Patch-Test (visuelle Bewertung der behandelten Haut)
o 44 Probanden
o 3 x 22 h Behandlung
o Negativkontrolle: aqua demin.
o Ergebnis (gefärbte Rezeptur):
   ■ Mittlerer Summenscore: 0.50
o Ergebnis (ungefärbte Rezeptur):
   ■ Mittlere Summenscore: 0.79
o Negativkontrolle:
   ■ Mittlere Summenscore: 0.92

Es zeigt sich, dass die Abweichung zwischen der lokalen Verträglichkeit der gefärbten gegenüber der ungefärbten Rezeptur wesentlich geringer ist als bei den entsprechenden Azo-Farbstoffen. Anhand der 44 Probanden konnte belegt werden, dass bei dem erfindungsgemäßen Hautfärbemittel, die lokale Verträglichkeit vergleichbar zur farbstofffreien Rezeptur ist.

## Patentansprüche

1. Antiseptisches Hautfärbemittel enthaltend
a. 0,00001 - 1 Gew.- % einen oder mehrere Farbstoffe,
b. 50 - 99 Gew.-% einen oder mehrere alkoholische Wirkstoffe mit 1 bis 3 Kohlenstoff-Atomen,
c. 0 - 5 Gew.-% eine oder mehrere hautverträgliche, organische Säuren,
d. 0,05 - 5 Gew.-% weitere Hilfsstoffe, wobei 0,05 bis 1 Gew.-% hiervon Polyvinylpyrrolidon (PVP) sind,
e 0 - 5 Gew.-% weitere Wirkstoffe und
f. Wasser,
wobei sich die Komponenten a. bis f. zu 100 Gew.-% ergänzen und der Farbstoff Chinolingelb (E 104), Patentblau V (E 131), Brillantgrün BS (E 142) und/oder Indigotin (E 132) ist.

2. Hautfärbemittel gemäß Anspruch 1, **dadurch gekennzeichnet, dass** der alkoholische Wirkstoff Ethanol, 1-Propanol, 2-Propanol oder eine Mischung hiervon ist.

3. Hautfärbemittel gemäß einem der vorhergehenden Ansprüche, **dadurch gekennzeichnet, dass** das Hautfärbemittel 50 bis 95 Gew. -%, bevorzugt 50 bis 70 Gew.-% und besonders bevorzugt 50 bis 65 Gew.-%, oder bevorzugt 75 - 87 Gew.-% alkoholischen Wirkstoff enthält.

4. Hautfärbemittel gemäß einem der vorhergehenden Ansprüche, **dadurch gekennzeichnet, dass** das Hautfärbemittel als Hilfsstoffe wasserlösliche und/oder alkohollösliche Polymere, Verdickungsmittel, Vergällungsmittel, Emulgatoren und/oder hautverträgliche Tenside enthält.

5. Hautfärbemittel gemäß einem der vorhergehenden Ansprüche, **dadurch gekennzeichnet, dass** das Hautfärbemittel als antimikrobielle Wirkstoffe ausschließlich Ethanol, 1-Propanol und /oder 2-Propanol enthält.

6. Hautfärbemittel gemäß einem der Ansprüche 1 bis 4, **dadurch gekennzeichnet, dass** das Hautfärbemittel weitere antimikrobiell wirksame Stoffe enthält.

7. Hautfärbemittel gemäß einem der vorhergehenden Ansprüche, **dadurch gekennzeichnet, dass** die hautverträgliche, organische Säure, eine Mono-, Di- oder Polycarbonsäure mit ggf. einer oder mehreren Hydroxygruppen ist oder bevorzugt Citronensäure, Ascorbinsäure, Milchsäure oder Pidolsäure, bevorzugt Milchsäure, ist.

8. Hautfärbemittel gemäß einem der vorhergehenden Ansprüche zur Anfärbung von Hautarealen vor einer chirurgischen oder medizinischen Behandlung oder/und als Hautdesinfektionsmittel.

## Claims

1. An antiseptic skin dye containing
a. 0.00001 - 1 wt.% one or more dyes,
b. 50 - 99 wt.% one or more alcoholic active substances with 1 to 3 carbon atoms,
c. 0 - 5 wt.% one or more skin-compatible, organic acids,
d. 0.05 - 5 wt.% other auxiliary substances, wherein 0.05 to 1 wt.% thereof are polyvinyl pyrrolidone (PVP),
e. 0 - 5 wt.% other active substances and
f. water,
wherein the components a. to f. add up to 100 wt.% and the dye is quinoline yellow (E 104), patent blue V (E 131), brilliant green BS (E 142) and/or indigotin (E 132).

2. The skin dye according to claim 1, **characterised in that** the alcoholic active substance is ethanol, 1-propanol, 2-propanol or a mixture thereof.

3. The skin dye according to one of the preceding claims, **characterised in that** the skin dye contains 50 to 95 wt.%, preferably 50 to 70 wt.% and particularly preferably 50 to 65 wt.%, or preferably 75 - 87 wt.% alcoholic active substance.

4. The skin dye according to one of the preceding claims, **characterised in that** the skin dye contains water-soluble and/or alcohol-soluble polymers, thickening agents, denaturing agents, emulsifiers and/or skin-compatible surfactants as auxiliary substances.

5. The skin dye according to one of the preceding claims, **characterised in that** the skin dye contains exclusively ethanol, 1-propanol and /or 2-propanol as antimicrobial active substances.

6. The skin dye according to one of claims 1 to 4, **characterised in that** the skin dye contains additional antimicrobially active substances.

7. The skin dye according to one of the preceding claims, **characterised in that** the skin-compatible organic acid is a mono-, di- or polycarboxylic acid with optionally one or more hydroxy groups or is preferably citric acid, ascorbic acid, lactic acid or pidolic acid, preferably lactic acid.

8. The skin dye according to one of the preceding claims for staining areas of skin before a surgical or medical treatment and/or as a skin disinfectant.

## Revendications

1. Agent antiseptique colorant la peau, contenant :
a) de 0,00001 à 1 % en poids d'un ou plusieurs colorant(s),
b) de 50 à 99 % en poids d'une ou plusieurs substance(s) active(s) de type alcool, comportant 1 à 3 atomes de carbone,
c) de 0 à 5 % en poids d'un ou plusieurs acide(s) organique(s) compatible(s) avec la peau,
d) de 0,05 à 5 % en poids d'autres adjuvants, dont 0,05 à 1 % en poids de polyvinylpyrrolidone (PVP),
e) de 0 à 5 % en poids d'autres substances actives,
f) et de l'eau,
étant entendu que les composants (a) à (f) font en tout 100 % en poids,
et dans lequel agent le colorant est du jaune de quinoléine (E 104), du bleu patenté V (E131), du vert brillant BS (E 142) et/ou de l'indigotine (E132).

2. Agent colorant la peau, conforme à la revendication 1, **caractérisé en ce que** la substance active de type alcool est de l'éthanol, du propane-1-ol ou du propane-2-ol, ou un mélange de ces alcools.

3. Agent colorant la peau, conforme à l'une des revendications précédentes, **caractérisé en ce que** cet agent colorant la peau contient de 50 à 95 % en poids, de préférence de 50 à 70 % en poids et en particulier de 50 à 65 % en poids, ou de préférence de 75 à 87 % en poids, de substance active de type alcool.

4. Agent colorant la peau, conforme à l'une des revendications précédentes, **caractérisé en ce que** cet agent colorant la peau contient, en tant qu'adjuvants, des polymères hydrosolubles et/ou des polymères solubles dans les alcools, des épaississants, des dénaturants, des émulsifiants et/ou des tensioactifs compatibles avec la peau.

5. Agent colorant la peau, conforme à l'une des revendications précédentes, **caractérisé en ce que** cet agent colorant la peau contient exclusivement, en tant que substances actives anti-microbiennes, de l'éthanol, du propane-1-ol et/ou du propane-2-ol.

6. Agent colorant la peau, conforme à l'une des revendications 1 à 4, **caractérisé en ce que** cet agent colorant la peau contient d'autres substances dotées d'une activité anti-microbienne.

7. Agent colorant la peau, conforme à l'une des revendications précédentes, **caractérisé en ce que** l'acide organique compatible avec la peau est un acide monocarboxylique, dicarboxylique ou polycarboxylique, éventuellement porteur d'un ou plusieurs groupe(s) hydroxyle, ou est de préférence de l'acide citrique, de l'acide ascorbique, de l'acide lactique ou de l'acide pidolique, en particulier de l'acide lactique.

8. Agent colorant la peau, conforme à l'une des revendications précédentes, servant à colorer des zones de la peau avant une opération chirurgicale ou un traitement médical et/ou servant d'agent désinfectant pour la peau.
